# EUROPEAN PATENT APPLICATION

(11) **EP 2 324 806 A1**
(43) Date of publication of application: **25.05.2011**
(21) Application number: 09813153.5
(22) Date of filing: 11.09.2009
(51) Int. Cl.: A61F 13/15, A61F 13/472, A61F 13/539

(54) **ABSORBENT ARTICLE**

(30) Priority: 12.09.2008 JP 2008235439
(71) Applicant: Unicharm Corporation, Ehime 799-0111 (JP)
(72) Inventor: SAGISAKA, Minako, Kagawa 769-1602 (JP); OKADA, Saori, Kagawa 769-1602 (JP); NISHITANI, Kazuya, Kagawa 769-1602 (JP); HAYASHI, Toshihisa, Kagawa 769-1602 (JP)
(74) Representative: Knights, Rupert
(86) International application number: PCT/JP2009/065954
(87) International publication number: WO 2010/030007

(57) **Abstract**

In the absorbent article 1 according to the present invention, each groove 10 of the pair of grooves in a central region in the longitudinal direction L has a first section 3A which is formed so as to be curved in a convex manner at an inside of a widthwise direction W of the absorption layer; a second section 3B and a third section 3C which is linked with each end of the first section 3A and is formed so as to be curved in a convex manner at an outside of the widthwise direction. A radius of curvature of the first section 3A is smaller than a respective one of a radius of curvature of the second section 3B and a radius of curvature of the third section 3C, and a virtual line V1 which connects a side surface 13B2 which is opposite to a side surface 13B1, 13C1 which forms an outermost part in the widthwise direction W in the second section 3B and a side face 13C2 which forms an outermost part in the widthwise direction W in the third section 3C, the virtual line V1 is adapted so as to be positioned at an outside of the widthwise direction W more than a side surface 13A2 which is opposite to a side surface 13A1 which forms an innermost part in the widthwise direction W in the first section 3A.

## Description

### [Technical Field]

The present invention relates to an absorbent article which is provided with an absorption layer.

### [Background Art]

Conventionally, there are known absorbent articles, each of which is capable of providing an excellent sense of wearing by providing a groove at a skin abutment face side of an absorption layer which is comprised of a topsheet and an absorber to thereby cause the absorber to rise toward a skin side when the article is worn by a user (for example, refer to Patent Documents 1 and 2).

### [Prior Art Document]

### [Patent Document(s)]

[Patent Document 1]
   Japanese Unexamined Patent Application Publication No. 2002-282304
[Patent Document 2]
   Japanese Unexamined Patent Application Publication No. 2006-325639

### [Problem(s) to Be Solved by the Invention]

However, since, in the conventional absorbent articles, a groove in a central region in a longitudinal direction of the abovementioned absorption layer is formed by a smooth curve, a bottom face which forms the groove hardly rises in a vertical direction, in response to a force from the outside in a widthwise direction of the absorption layer in the central region, which is generated when the absorbent articles are worn by users.

Therefore, in the conventional absorbent articles, there has been a problem that, when the absorbent articles are worn by users, even in a case where a force from the outside in a widthwise direction of the absorption layer is generated in the central region, it is difficult to support an absorber which has risen on a skin side by means of a bottom face which forms the groove; the absorber is readily restored to a state before the bottom face rises, by means of a force from a skin side; a gap is readily generated between a user's body and the absorber; and leakage is likely to occur.

Therefore, the present invention has been made in view of the above-described problem, and it is an object of the present invention to provide an absorbent article which is capable of supporting an absorber which has risen on a skin side, by means of a bottom face which forms a groove which is provided on a skin abutment face side of an absorption layer when the absorbent article has been worn by a user.

### [Means for Solving the Problem(s)]

The first feature of the present invention is summarized in that an absorbent article, including an absorption layer, wherein a pair of grooves are provided at a side edge on a skin abutment face side of the absorption layer, the pair of grooves are formed in a substantially line symmetric shape relative to a centerline which extends in a longitudinal direction of the absorption layer, each groove of the pair of grooves in a central region in the longitudinal direction has: a first section which is formed so as to be curved in a convex manner at an inside of a widthwise direction of the absorption layer; a second section and a third section which is linked with each end of the first section and is formed so as to be curved in a convex manner at an outside of the widthwise direction, the first section is disposed at a center of the central region, a radius of curvature of the first section is smaller than a respective one of a radius of curvature of the second section and a radius of curvature of the third section, and a virtual line which connects a side surface which is opposite to a side surface which forms an outermost part in the widthwise direction in the second section and a side face which forms an outermost part in the widthwise direction in the third section, the virtual line is adapted so as to be positioned at an outside of the widthwise direction more than a side surface which is opposite to a side surface which forms an innermost part in the widthwise direction in the first section.

### [Advantageous Effect(s) of the Invention]

As has been described above, according to the present invention, an absorbent article can be provided which is capable of supporting an absorber which has risen on a skin side, by means of a bottom face which forms a groove which is provided on a skin abutment face side of an absorption layer when the absorbent article has been worn by a user.

### [Brief Description of Drawings]

[Fig. 1] Fig. 1 is a plan view which is seen from a topsheet side of an absorbent article according to a first embodiment of the present invention.
[Fig. 2] Fig. 2 is an enlarged view of a bottom face of a groove which is provided at a skin abutment face side of an absorption layer of the absorbent article according to the first embodiment of the present invention.
[Fig. 3] Fig. 3 is a view showing a shape in a central region of the groove of the absorption layer of the absorbent article according to the first embodiment of the present invention.
[Fig. 4] Fig. 4 is a plan view which is seen from a surface sheet side of an absorbent article according to Exemplary Modification 1 of the present invention.

### [Best Mode for Carrying out the Invention]

### (Configuration of Absorbent Article According to First Embodiment of the Present Invention)

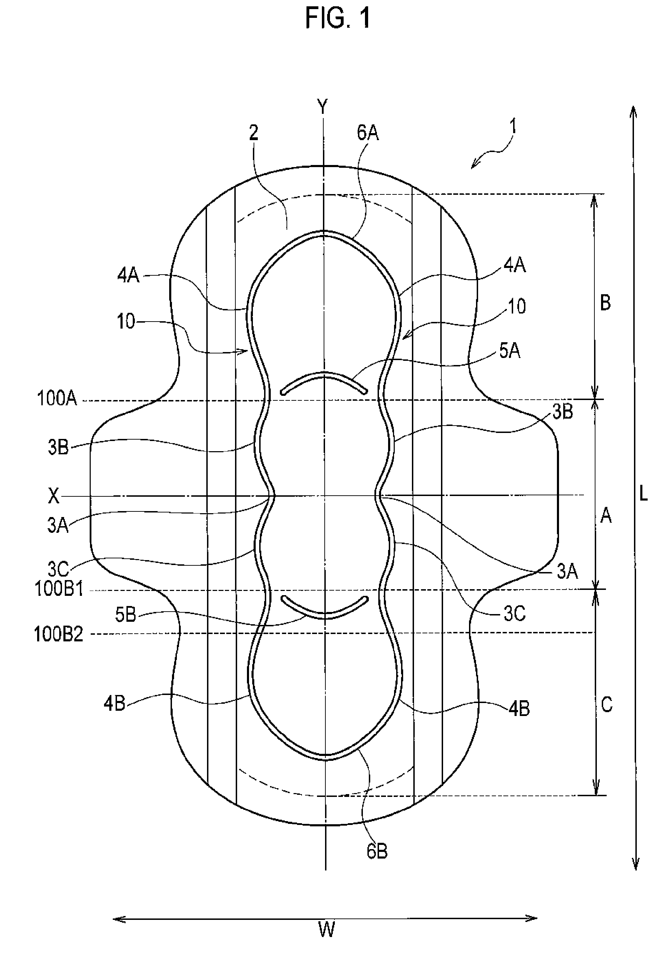

Referring to Fig. 1 to Fig. 3, a configuration of an absorbent article according to a first embodiment of the present invention will be described. Fig. 1 is a plan view when an absorbent article 1 according to the embodiment is seen from a topsheet side (a user's skin abutment side).

While, in the embodiment, a napkin for daytime is described as a typical example of the absorbent article 1 according to the present invention, the present invention is assumed to be applicable to a napkin for nighttime, an incontinence pad, a diaper, or a panty liner or the like, without being limitative thereto.

The absorbent article 1 according to the embodiment is provided with: a liquid-permeable topsheet; a liquid-impermeable backsheet; an absorber which is disposed between the topsheet and the backsheet; and a flap portion which is comprised of a nonwoven cloth and is formed to extend laterally from both edges. The flap portion shown in the embodiment may be absent.

In the conventional techniques, the absorbent article according to the present invention may be manufactured by means of a combination of known techniques related to these conventional techniques. For example, the topsheet, the backsheet, and the absorber may be connected to the absorbent article by means of thermal deposition, hot melting, or any other manufacturing technique which is analogous thereto.

For example, the topsheet may be constituted by means of a woven cloth, which is comprised of a natural fiber or a chemical fiber, and/or a nonwoven cloth or a combination therebetween. Here, examples of natural fibers can include those which include celluloses such as crushed pulp or cotton. In addition, examples of chemical fibers can include: reproductive celluloses such as rayon or fibril rayon; semisynthetic celluloses such as acetate or triacetate; thermoplastic hydrophobic chemical fibers; or thermoplastic hydrophobic chemical fibers to which hydrophilization treatment is applied. Further, examples of thermoplastic hydrophobic chemical fibers can include: single fibers such as polyethylene (PE), polypropylene (PP), or polyethylene terephthalate (PET); fibers which are formed by graft-polymerizing PE and PP; or composite fibers of core-sheath structures or the like.

Furthermore, it is preferable that: the absorber is made of pulp, chemical pulp, rayon, acetate, natural cotton, polymeric absorber, fiber-like polymeric absorber, synthetic resin or foam or the like solely or a mixture of these materials; and that the absorber is the one that hardly loses its shape and has less chemical stimulation.

Here, the absorber may be comprised of a single-layered absorber or may be comprised of a multi-layered absorber. In addition, a sheet such as a cushion sheet or a dispersion sheet may be disposed between the topsheet and the absorber.

It is assumed that an absorption layer 2 is comprised of the topsheet and the absorber (including the above sheet).

Further, it is preferable that the backsheet is comprised of polyethylene, polypropylene, polyethylene terephthalate, polyvinyl alcohol, polylactate, polybutyl succinate, nonwoven cloth, or paper of which thickness is 15 microns to 60 microns, or alternatively, a laminate material thereof. Furthermore, the backsheet may be comprised of a ventilation film which is obtained by filling with an inorganic filler, and then, applying a stretching process thereto.

As shown in Fig. 1, one pair of grooves 10, 10 are provided at a side edge of a skin abutment face side of the absorption layer 2 in the absorbent article 1 according to the embodiment. In addition, such one pair of grooves 10, 10 are formed in a substantially line-symmetric shape relative to a centerline Y which extends to a longitudinal direction L of the absorption layer 2.

In addition, the absorbent article 1 according to the embodiment has: a central region A taken along the longitudinal direction L of the absorption layer 2; and an anterior region B and a posterior region C which are provided at the outside of the longitudinal direction L of the central region A.

Here, the central region A is a region which includes a centerline X which extends in a widthwise direction W of the absorption layer 2 or a region which includes a portion abutting against an excretion portion. The central region A is positioned between two folds 100A and 100B2 when the absorbent article 1 is folded into three sections and is individually packed. Specifically, the central region A is a region which is defined by a line 100B1, which extends in the widthwise direction W of the absorption layer 2, and a fold 100A. While, in the embodiment, the central region A is positioned between two folds 100A and 100B2 when the absorbent article 1 is folded into three sections and is individually packed, the folds may be positioned in different locations or may be absent without being formed.

In addition, each groove of one pair of grooves 10, 10 in the central region A in the longitudinal direction L has: a first section 3A which is formed so as to be curved in a convex manner at the inside of the widthwise direction W of the absorption layer 2; and a second section 3B and a third section 3C which are linked with both ends of the first section 3A and are formed so as to be curved in a convex manner at the outside of the widthwise direction W. That is, each of the grooves 10 has the first section 3A, the second section 3B, and the third section 3C, a respective one of which is continuously formed.

In addition, each groove of one pair of grooves 10, 10 in the anterior region B and the posterior region C has a fourth section 4A and a fifth section 4B which are formed so as to be curved in a convex manner at the outside of the widthwise direction W of the absorption layer 2, respectively. While, in the embodiment, the fourth section 4A and the fifth section 4B are shown, the fourth section 4A and the fifth section 4B may be absent. If neither the fourth section 4A nor the fifth section 4B is present, such each of the grooves 10 is comprised of the first section 3A, the second section 3B, and the third section 3C.

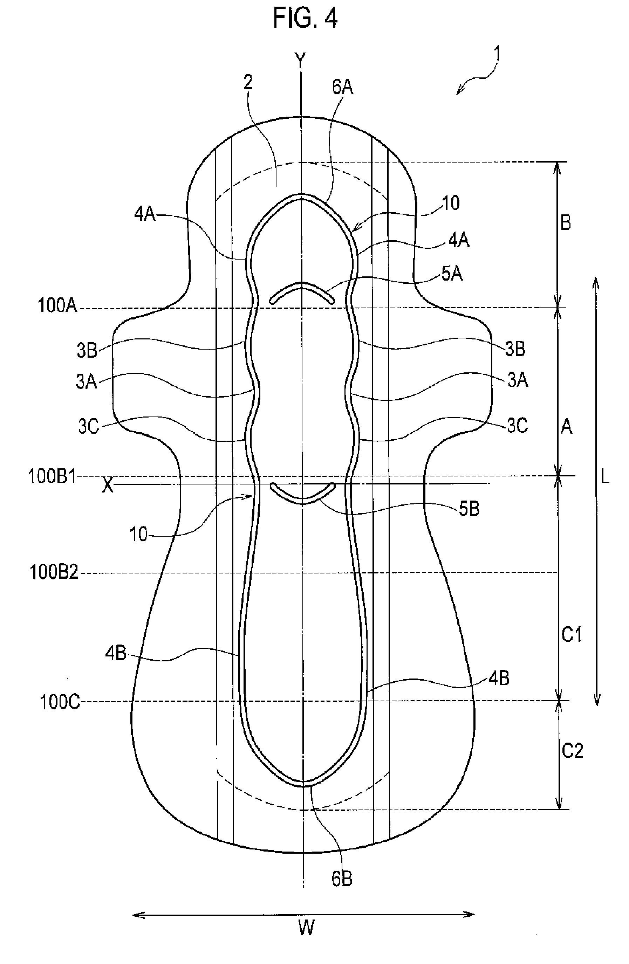

Here, the first section 3A is disposed at a center of the central region A. In the embodiment, the central region A is positioned on the centerline X, whereas in another embodiment, for example, as shown in a second embodiment of Fig. 4, the center of the central region A may not be positioned on the centerline X. In addition, the second section 3B and the third section 3C are formed in a substantially line-symmetric shape relative to a centerline which extends in the widthwise direction W of the first section 3A that is positioned on the centerline X in the embodiment. While, in the embodiment, the second section 3B and the third section 3C are formed in a substantially line-symmetric shape relative to the centerline X that extends in the widthwise direction W of the first section 3A, the second section 3B and the third section 3C may be formed in different shapes.

In addition, a radius of curvature of the first section 3A is smaller than a respective one of those of the second section 3B and the third section 3C; the radius of curvature of the second section 3B and the radius of curvature of the third section 3C are substantially identical to each other; and the radiuses of curvatures of the fourth section 4A and the fifth section 4B are greater than those of the second section 3B and the third section 3C. While, in the embodiment, the radius of curvature of the second section 3B and the radius of curvature of the third section 3C are substantially identical to each other, the radius of curvature of the second section 3B and the radius of curvature of the third section 3C may be different from each other.

Further, grooved portions 5A, 5B, 6A, 6B which extend in the widthwise direction W are formed at the outside of the longitudinal direction L of the second section 3B and the third section 3C. That is, the grooved portions 5A, 5B, 6A, 6B are disposed at the outside of the longitudinal direction L in the central region A. Neither the left nor right ends of at least one of the grooved portions 5A, 5B, 6A, 6B is linked with one pair of grooves 10, 10. That is, both of the left and right ends of at least one of the grooved portions 5A, 5B, 6A, 6B are spaced from one pair of grooves 10, 10. In an example of Fig. 1, both of the left and right ends of the grooved portions 5A, 5B are spaced from one pair of grooves 10, 10. As another example, both of the left and right ends of the grooved portions 6A, 6B are spaced from one pair of grooved 10, 10. While, in the embodiment, the grooved portions 5A, 5B, 6A, 6B are shown, the grooved portions 5A, 5B and/or the grooved portions 6A, 6B may be absent.

Furthermore, the second section 3B and the fourth section 4A are linked with each other by means of part of the groove 10 that is formed so as to be curved to the inside of the widthwise direction W, and the third section 3C and the fifth section 4B are linked with each other by means of part of the groove 10 that is formed so as to be curved to the inside of the widthwise direction W.

Still furthermore, in a case where a length in the longitudinal direction L of the absorber is defined to be a typical length (for example, 140 mm to 440 mm), it is preferable that a length in the longitudinal direction L of the center region A is 40 mm or more, and it is further preferable that the length is 60 mm to 120 mm.

Yet furthermore, in a case where a length in the longitudinal direction L of the absorber is defined to be a typical length (for example, 40 mm to 100 mm), it is preferable that a length in the longitudinal direction L of the center region A is 10 mm or more, and it is further preferable that the length is 25 mm to 50 mm.

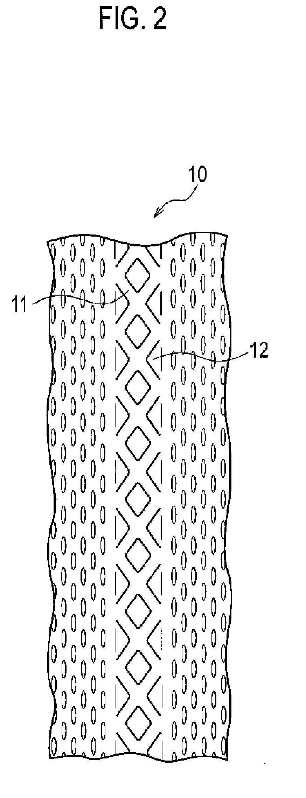

As shown in Fig. 2, a high pressure compression portion 12 and a low pressure compression portion 11 which forms a step difference lower than the high pressure compression portion 12 are provided on a bottom face which forms one pair of grooves 10, 10, and the low pressure compression portion 11 is constituted so as to be continuous. For example, the low pressure compression portion 11, as shown in Fig. 2, is constituted so as to be disposed continuously in a lattice shape. The high pressure compression portion 12 and the low pressure compression portion 11 are provided in a process of forming one pair of grooves 10, 10. It is preferable that one pair of grooves 10, 10 are formed by means of emboss processing which is a known technique. In the high pressure compression portion 12, a topsheet and an absorber are compressed to be higher than those in low pressure compression portion 11, and are regions in which a fiber constituting the topsheet and the absorber has a high density than that of the low pressure compression portion 11. The high pressure compression portion 12 and the low pressure compression portion 11 may be formed by employing a roll having a pattern for performing emboss processing. In this case, the abovementioned roll is provided with: a planar region which is formed along a circumferential direction of the roll; and a convex region which bulges in a radial direction of the roll more than the planer region. The high pressure compression portion 12 is formed by means of the convex region in the abovementioned roll. The low pressure compression portion 11 is formed by means of the planar region. While, in the embodiment, the high pressure compression portion 12 and the low pressure compression portion 11 are provided on the bottom face which forms one pair of grooves 10, 10, one pair of grooves 10, 10 may be formed to be continuously flat by means of emboss processing. In addition, the high pressure compression portion 12 and the low pressure compression portion 11 may be formed in different patterns, respectively.

With the absorbent article 1 according to the first embodiment of the present invention, it is preferable that: the low pressure compression portion 11 is continuous; and that a region in which a groove is not provided in the absorption layer 2 is not adjacent to the high pressure compression portion 12.

In such a case, when one pair of grooves 10, 10 are formed by means of a roll, a portion at which a difference of elevation is extremely great is a few in number, and the topsheet hardly breaks on a boundary between one pair of grooves 10, 10 and a region in which a groove is not provided in the absorption layer 2.

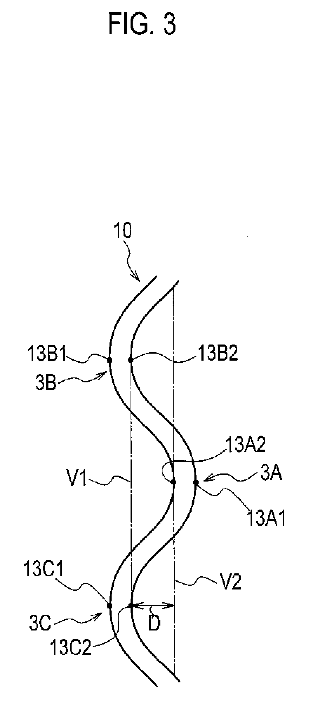

In addition, as shown in Fig. 3, a virtual line V1 which connects a side surface 13B2, which is opposite to a side surface 13B1 forming the outermost part in the widthwise direction W in the second section 3B, and a side surface 13C2, which is opposite to a side surface 13C1 forming the outermost part in the widthwise direction W in the third section 3C, is adapted so as to be positioned at the outside of the widthwise direction W more than a side surface 13A2, which is opposite to a side surface 13A1 forming the innermost part in the widthwise direction W in the first section 3A.

That is, each groove of one pair of grooves 10, 10 has: an inner side surface (inside edge) and an outer side surface (outside edge) in a widthwise direction. Here, inner side surfaces of the second section 3B and the third section 3C at the apexes of a curve protruding to the outside of the widthwise direction W are positioned at the outside of the widthwise direction W more than the outer side surface in the widthwise direction W of the first section 3A.

Here, in a case where the absorbent article 1 has been worn by a user, the following phenomena will arise at the first section 3A, the second section 3B, and the third section 3C that have been thus constituted.

First, when the absorbent article 1 has been worn by a user, a force from the outside in the widthwise direction W is applied to the side surface 13B1 and the side surface 13C1 mentioned above.

Second, the force that is applied to the abovementioned side surface 13B1 and side surface 13C1 is transmitted into the groove 10 toward the inside in the widthwise direction W.

Third, a force oriented to the outside in the widthwise direction, which is generated from a risen absorber and a force oriented to the inside in the widthwise direction W after transmitted into the groove 10, are applied to the first section 3A.

Fourth, in the first section 3A, a distortion arises at a site at which rigidity relative to these forces is low, and with the site being a starting point, a bottom face which forms the first section 3A rises in a vertical direction.

On the other hand, even in a case where the absorbent article 1 has been worn by a user, a bottom face which forms the second section 3B and the third section 3C is left to have been maintained in a horizontal state without rising in a vertical direction.

It is preferable that a distance D between the abovementioned virtual straight line V1 and a tangential line V2 on a side surface 13A2 which is opposite to a side surface 13A1 forming the innermost part in the widthwise direction W in the first section 3A is 0.5 mm or more and less than 15 mm.

In this manner, in the first section 3A, the rigidity relative to the abovementioned forces is lowered, and the bottom face which forms the first section 3A readily rises in a vertical direction.

In addition, it becomes easy to adjust a starting point at which the bottom face which forms the first section 3A rises in a vertical direction.

As a result, in a case where a position at which a user wears underwear of the absorbent article 1 is shifted at the left and right, a starting point at which the bottom face which forms the first section 3A rises in a vertical direction is never shifted at the left and right, and thereafter, an optimal sense of wearing can be provided.

In this manner, in a case where a user has taken a seat, it is possible to avoid a failure that the first section is folded and bent due to a force which is applied from a user's body to a rising absorber.

According to the absorbent article 1, it is preferable that the central region A is positioned between two folds 100A and 100B2 when the absorbent article 1 is folded up.

Accordingly, a rise on a skin abutment face side of the absorber in the central region A is never impeded due to winkles of the folds 100A, 100B2.

According to the absorbent article 1, it is preferable that: the radius of curvature of the second section 3B and the radius of curvature of the third section 3C are substantially identical to each other; and that the second section 3B and the third section 3C are formed in a substantially line-symmetric shape relative to the centerline X.

Accordingly, a rise of the first section 3A becomes substantially line symmetric relative to the centerline X and then a rise against the skin abutment face side of the absorber in the central region A also becomes substantially line-symmetric relative to the centerline X, whereby the rise on the skin abutment face side of the absorber in the central region A can be stabilized.

According to the absorbent article 1, it is preferable that: the high pressure compression portion 12 and the low pressure compression portion 11 are provided on the bottom face which forms one pair of grooves 10, 10; and that the low pressure compression portion 11 is constituted to be continuous.

Accordingly, while one pair of grooves 10, 10 maintain flexibility faithfully following a body, rigidity is improved and deformation can be stabilized.

According to the absorbent article 1, it is preferable that each groove of one pair of grooves 10, 10 in the anterior region B and the posterior region C has the fourth section 4A and the fifth section 4B that are formed to be curved in a convex manner at the outside of the widthwise direction W.

Accordingly, since the absorbers in the anterior region B and the posterior region C also rise, like the absorber in the central region A, in case that the menstrual blood that could not be absorbed by the absorber in the central region A has flown in a forward/backward direction, the leakage of the menstrual blood can be prevented by the absorbers in the anterior region B and the posterior region C.

According to the absorbent article 1, it is preferable that the radiuses of curvatures of the fourth section 4A and the fifth section 4B are greater than those of the second section 3B and the third section 3C.

Accordingly, one pair of grooves 10, 10 broaden to the outside in the widthwise direction as they run in the forward/backward direction of the longitudinal direction L, and a shape taken along the shape of the inguinal region of legs can be formed.

According to the absorbent article 1, it is preferable that the grooved portions 5A, 5B, 6A, 6B which extend in the widthwise direction W are formed at the outside of the longitudinal direction L of the second section 3B and the third section 3C.

Accordingly, in a case where menstrual blood has flown in the forward/backward direction of the longitudinal direction L while the blood cannot be absorbed by the absorber in the central region A, the menstrual blood can be stopped by the grooved portions 5A, 5B, 6A, 6B which are high density regions.

In addition, according to the central region A, a user is allowed to clearly visually recognize the central region A, so that a displacement of wearing can hardly occur.

According to the absorbent article 1, it is preferable that these grooved portions 5A, 5B, 6A, 6B are positioned at the outside of the longitudinal direction L in the central region A.

Accordingly, it is possible to avoid a failure that a rise of the absorber in the central region A is impeded.

According to the absorbent article 1, it is preferable that both of the left and right ends of the grooved portions 5A, 5B, 6A, 6B are spaced from one pair of grooves 10, 10.

Accordingly, the absorber in the central region A is allowed to rise on the skin abutment face side without an occurrence of a kink, and leakage of bodily fluid can be prevented.

### (Exemplary Modification 1)

Referring to Fig. 4, an absorbent article 1 according to Exemplary Modification 1 of the present invention will be described. While the aforementioned first embodiment described an example in which a napkin for daytime is enumerated as the absorbent article 1, this Exemplary Modification 1 will describe an example in which a napkin for nighttime is enumerated as the absorptive article 1, focusing on differences from the absorbent article 1 according to the foregoing first embodiment.

As shown in Fig. 4, the absorbent article 1 according to this Exemplary Modification 1 may be constituted so as to be folded up by means of three folds 100A, 100B2, 100C, and then, be individually packed. In such a case, the central region A may be positioned between two folds 100A and 100B2.

While the present invention was described in detail by employing the aforementioned embodiments, it is apparent that the present invention is not limitative to the embodiments described in the specification. The present invention can be implemented as alteration and modification aspects without deviating from the gist and scope of the present invention that is defined by the recitations of the claims that follow. Therefore, the descriptive matters of the specification are intended to furnish illustrative explanation and do not have any limitative meaning to the present invention.

The entire contents of Japanese Patent Application No. 2008-235439 (filed on September 12, 2008) are incorporated in the specification of the present application by reference.

### [Industrial Applicability]

As described above, the absorbent article according to the present is capable of supporting an absorber which has risen on a skin side, by means of a bottom face which forms a groove which is provided on a skin abutment face side of an absorption layer when the absorbent article has been worn by a user, it is valuable for an absorbent article such as a napkin, an incontinence pad, a diaper, or a panty liner or the like.

## Claims

1. An absorbent article, comprising an absorption layer, wherein
a pair of grooves are provided at a side edge on a skin abutment face side of the absorption layer,
the pair of grooves are formed in a substantially line symmetric shape relative to a centerline which extends in a longitudinal direction of the absorption layer,
each groove of the pair of grooves in a central region in the longitudinal direction has: a first section which is formed so as to be curved in a convex manner at an inside of a widthwise direction of the absorption layer; a second section and a third section which is linked with each end of the first section and is formed so as to be curved in a convex manner at an outside of the widthwise direction,
the first section is disposed at a center of the central region,
a radius of curvature of the first section is smaller than a respective one of a radius of curvature of the second section and a radius of curvature of the third section, and
a virtual line which connects a side surface which is opposite to a side surface which forms an outermost part in the widthwise direction in the second section and a side face which forms an outermost part in the widthwise direction in the third section, the virtual line is adapted so as to be positioned at an outside of the widthwise direction more than a side surface which is opposite to a side surface which forms an innermost part in the widthwise direction in the first section.

2. The absorbent article according to claim 1, wherein the central region is positioned between two folds when the absorbent article is folded up.

3. The absorbent article according to claim 1 or 2, wherein
a radius of curvature of the second section and a radius of curvature of the third section are substantially identical to each other, and
the second section and the third section are formed in a substantially line-symmetric shape relative to a centerline which extends in the widthwise direction of the first section.

4. The absorbent article according to any one of claims 1 to 3, wherein,
a high pressure compression portion and a low pressure compression portion which forms a step difference lower than the high pressure compression portion are provided on a bottom face which forms the pair of grooves, and
the low pressure compression portion is constituted so as to be continuous.

5. The absorbent article according to any one of claims 1 to 4, wherein
the absorbent article has an anterior region and a posterior region at an outside of the longitudinal direction in the center region,
each groove of the pair of grooves in the anterior region and the posterior region has a fourth section and a fifth section which are formed so as to be curved in a convex manner at an outside of a widthwise direction of the absorption layer, respectively.

6. The absorbent article according to claim 5, wherein
the radiuses of curvatures of the fourth section and the fifth section are greater than radiuses of curvatures of the second section and the third section.

7. The absorbent article according to any one of claims 1 to 6, wherein
a grooved portion extending in the widthwise direction is formed at an outside of the longitudinal direction of the second section and the third section.

8. The absorbent article according to claim 7, wherein both of left and right ends of the grooved portion are spaced from the pair of grooves.
